# EUROPÄISCHE PATENTSCHRIFT

(11) **EP 2 536 841 B1**
(45) Veröffentlichungstag und Bekanntmachung des Hinweises auf die Patenterteilung: **20.04.2016**
(21) Anmeldenummer: 11721220.9
(22) Anmeldetag: 17.02.2011
(51) Int. Cl.: C12P 7/06, C12P 19/12, A23K 30/15

(54) **VERWENDUNG EINES SILIERMITTELS ZUR BEHANDLUNG VON UNZERKLEINERTEN RÜBEN**
USE OF AN ENSILING AGENT TO TREAT WHOLE TAPROOTS
UTILISATION D'UN PRODUIT D'ENSILAGE POUR LE TRAITEMENT DE BETTERAVES NON FRAGMENTÉES

(30) Priorität: 18.02.2010 DE 102010008516
(43) Veröffentlichungstag der Anmeldung: 26.12.2012
(73) Patentinhaber: KWS SAAT SE, 37574 Einbeck (DE)
(72) Erfinder: VON FELDE, Andreas, 29614 Soltau (DE)
(86) Internationale Anmeldenummer: PCT/DE2011/000142
(87) Internationale Veröffentlichungsnummer: WO 2011/100956

(56) Entgegenhaltungen:
- DE-A1- 2 404 462
- Mt Wu ET AL: "Control of Sucrose Loss in Sugarbeet During Storage by Chemicals1 and Modified Atmosphere and Certain Associated Physiological Changes", journal of the ASSBT, 15. Januar 1970 (1970-01-15), Seiten 117-127, XP55004799, Gefunden im Internet: URL:http://assbt-jsbr.org/JSBR/Vol16/JSBRV ol16No2P117to127ControlofSucroseLossinSuga rbeetDuringStoragebyChemicalandModifiedAtm osphereandCertainAssociatedPhsiologicalCha nges.pdf [gefunden am 2011-08-15]
- Wr Akeson ET AL: "Effect of Chemicals on Sucrose Loss in Sugarbeets During Storage", journal of the ASSBT, 15. August 1978 (1978-08-15), Seiten 255-268, XP55004801, Gefunden im Internet: URL:http://assbt-jsbr.org/JSBR/Vol20/JSBRV ol20No3P255to268EffectofChemicalsonSucrose LossinSugarbeetsDuringStorage.pdf [gefunden am 2011-08-15]
- LAUBE W ET AL: "STUDIES ON THE PRESERVATION OF TUBERS BY THE PROCESS OF ENSILAGE I ENSILAGE OF SUGAR BEETS-D WITH THE ADDITION OF DIFFERENT PRESERVATIVES", ARCHIV FUER TIERERNAEHRUNG, vol. 18, no. 3, 1968, pages 229-238, ISSN: 0003-942X

## Beschreibung

Die Erfindung betrifft die Verwendung eines Siliermittels zur Behandlung von unzerkleinerten Rüben. Darüber hinaus betrifft die Erfindung ein Verfahren zur Zucker- und Bioethanolgewinnung aus Zuckerrüben.

Rüben im Sinne dieser Anmeldung sind alle Pflanzen der Art *Beta vulgaris.* Hierzu gehören zum Beispiel Rote Rüben, Zucker- und Futterrüben sowie Mangold.

Zuckerrüben dienen grundsätzlich zur Zuckergewinnung. Hierzu werden sie geerntet und in speziellen Zuckerfabriken verarbeitet. Darüber hinaus sind Zuckerrüben als Substrat für die Biogas- und Bioethanolproduktion von Bedeutung.

Das Problem insbesondere bei der Verwertung von Zuckerrüben besteht jedoch darin, dass diese Rüben aufgrund lokaler klimatischer Bedingungen in der Regel nicht ganzjährig geerntet werden können. In vielen Anbaugebieten werden Rüben im Herbst eines Jahres geerntet, sie können jedoch nicht alle zur gleichen Zeit verarbeitet werden. So wird zum Beispiel ungefähr die Hälfte der in Deutschland verarbeiteten Zuckerrüben vor der Verarbeitung gelagert. Dies geschieht zum einen direkt auf dem Gelände einer Zuckerfabrik, der größte Teil der Rüben wird aber dezentral in Feldrandmieten gelagert. Die Lagerung erfolgt aerob.

Diese Lagerungsform geernteter Zuckerrüben ist aber problematisch, weil insbesondere verletzte Rüben während der Lagerung nicht unerhebliche Mengen ihres gespeicherten Zuckers (Saccharose) verlieren. Als primäre Saccharose-Abbauprodukte enstehen insbesondere Glukose und Fruktose, die nachfolgend teilweise weiter metabolisiert werden. Dies führt insgesamt zu einem deutlich verminderten Zuckergehalt von Lagerrüben, eine wirtschaftliche Weißzucker- oder Ethanolproduktion ist bei längerer Lagerdauer daher nicht gegeben. Die resultierenden "Verarbeitungskampagnen" für Zuckerrüben (in Deutschland ca. 100 Tage) begrenzen sehr maßgeblich die Wettbewerbsfähigkeit der Weißzucker- und Ethanolproduktion aus Zuckerrüben. Man hat daher versucht, geerntete Zuckerrüben z. B. in Folienschläuchen zu silieren.

Der Prozess der Silierung wird in unterschiedliche Phasen unterteilt: Nach Einlagerung des Pflanzenmaterials und Luftabschluss wird der im Silo enthaltene Restsauerstoff schnell veratmet. Unter dann anaroben Bedingungen kommt es auf der Substratoberfläche zur raschen Vermehrung von Mikroorganismen, insbesondere Milchsäurebakterien und Hefen, die die verfügbaren wasserlöslichen Kohlenhydrate (Mono- und Oligosaccharide) zu organischen Säuren und Alkohol abbauen. Im Inneren des Pflanzenmaterials bauen endogene Pflanzenenzyme Kohlenhydrate ebenfalls zu Säuren und Alkoholen ab. Durch den hierbei resultierenden pH-Wert-Abfall wird das Wachstum von unerwünschten Mikroorganismen und die Aktivität endogener Pflanzenenzyme begrenzt, es entsteht eine stabile Silage.

Gemäß dem Stand der Technik sind zur Silierung eine sehr feine Häckselung und hohe mechanische Verdichtung des Pflanzenmaterials notwendig, um
1. möglichst wenig Lufteinschluss zu erzielen, damit schnell anaerobe Bedingungen geschaffen werden können,
2. durch Zerstörung von Zellstrukturen Kohlenhydrate für Mikroorganismen besser verfügbar zu machen und
3. eine schnelle Diffusion von Säuren und damit eine pH-Wert-Absenkung im Pflanzenmaterial zu erreichen.

Insgesamt entstehen aber auch bei der Silierung hohe Zuckerverluste, zumal der Silierprozess nicht nur an der Oberfläche der Rüben stattfindet, sondern die Rüben "durchsilieren". Der Zucker wird also über den gesamten Rübenquerschnitt abgebaut.

Aus DE-OS 2 404 462 ist ein Verfahren zur Konservierung des Zuckergehaltes von aerob gelagerten Zuckerrüben bekannt, das den Einsatz von verdünnter Kalkmilch als "Desinfektionsmittel" vorsieht. Gebrannter Kalk (CaO) wird hierzu auf die Zuckerrüben aufgesprüht, oder die Rüben werden durch ein Kalkmilchbad geführt. Dadurch erhalten die Zuckerrüben einen desinfizierenden "Überzug", der unter aeroben Bedingungen an Wundstellen Zucker-Abbauprozesse verringern soll.

Ein weiteres Verfahren zur Konservierung von Zuckerrüben wird mit DD 226 764 A1 vorgeschlagen, wonach Silosickersaft anstelle bakterizid oder fungizid wirkender Chemikalien zur Silierung vorgesehen ist. Dieses Verfahren beinhaltet die Lagerung von vorzugsweise frisch geschnitzelten Rüben in der Konservierungsflüssigkeit in nicht ablaufenden Silos ohne Luftabschluss. Ein vollständiges Bedecken der Rüben mit Konservierungsflüssigkeit ist notwendig.

Auch eine Silierung von fein zerkleinerten Zuckerrüben für Futterzwecke ist beschrieben. Um Nährstoffverluste zu verringern, sieht DD 59 467 vor, die zerkleinerten Zuckerrüben mit Natriumbenzoat als Konservierungsmittel zu behandeln. Mit diesem Verfahren soll die Alkohol- und Essigsäuregärung eingeschränkt und das Abfließen von Sickersaft verhindert werden können.

Laube et al. (Laube, W.; Weissbach, F.; Budizer, H. H.: Untersuchungen zur Konservierung von Hackfrüchten durch Silierung, 1. Mitteilung: Die Silierung von Zuckerrüben unter Zusatz verschiedener Konservierungsmittel. In: Archiv für Tierernährung, Bd. 18, Heft 3, 1968, Seiten 229-238. ISSN: 003-942x) beschreiben ebenfalls die Silierung fein geschnitzelter Zuckerrüben mit Natriumbenzoat als Konservierungsmittel. Das Konservierungsmittel wurde der Silage beigemischt. Nach einer 6-monatigen Lagerdauer der Silage zeigten die behandelten Rübenschnitzel eine gegenüber der unbehandelten Kontrolle erhöhten Gehalt an Gesamtzucker. Über den Erhalt von Saccharose und den relativen Erhalt des Gesamtzuckers werden jedoch keine Angaben gemacht.

Während eine Silierung von zerkleinerten und unzerkleinerten Zuckerrüben vorteilhaft für eine anschließende Biogasgewinnung sein kann, weil sich silierte Zuckerrüben gut weiter vergären lassen, ist die Silierung als Vorstufe zur Zuckergewinnung oder Ethanolproduktion unerwünscht, denn bei der herkömmlichen Silierung verliert die Zuckerrübe erhebliche Mengen an Zucker. Dieser Zuckerverlust ist nicht auf einen Saccharoseverlust beschränkt. Auch Glukose und Fruktose gehen verloren.

Aufgabe der vorliegenden Erfindung ist es daher, die Lagerung von Rüben, insbesondere von Zuckerrüben, weiter zu vereinfachen und zu verbessern und damit auch eine Möglichkeit zu schaffen, die Ausbeute bei der Zucker- und Bioethanolgewinnung aus gelagerten Zuckerrüben zu steigern.

Erfindungsgemäß erfolgt die Lösung der gestellten Aufgabe durch die Verwendung eines Siliermittels zur Behandlung von unzerkleinerten Rüben, um bei einer Lagerung der Rüben einen Abbau von Zucker (Saccharose) zu reduzieren. Die Behandlung unzerkleinerter Rüben mit einem Siliermittel hat gegenüber der Behandlung von zerkleinerten Rüben den weiteren Vorteil, dass weniger Sickersaft entsteht, der technisch aufwändig abgeführt werden muss und dass die so behandelten Rüben problemlos handhabbar sind.

Überraschender Weise wurde gefunden, dass durch äußere Applikation eines Siliermittels auf unzerkleinerte Rüben die Zuckerabbauprozesse von der Rinde bis ins Zentrum des Rübenkörpers deutlich vermindert werden. Im Vergleich zu unbehandelten Kontrollen wird in den behandelten Rüben bei vergleichbarer pH-Wert-Erniedrigung deutlich weniger Zucker zu organischen Säuren und Alkoholen abgebaut. Sowohl Saccharose- als auch Gesamtzuckergehalt bleiben nach erfindungsgemäßer Behandlung hoch. Allerdings ist der Mechanismus der Signaltransduktion von der Rübenoberfläche bis in die inneren Bereiche des Rübenkörpers hierbei jedoch unbekannt.

Als unzerkleinerte Rüben im Sinne dieser Anmeldung werden auch solche Rüben verstanden, die während der Ernte oder anschließender Behandlung von bestimmten Frucht- oder Pflanzenteilen befreit werden. So werden Zuckerrüben bei der Ernte häufig geköpft oder entblättert. Der verbleibende Rübenkörper gilt dann als unzerkleinerte Rübe.

Als Siliermittel kommen im Grunde alle bekannten Formiat und/oder Propionat enthaltende Siliermittel in Betracht. Geeignete Siliermittel umfassen daher biologische und chemische Siliermittel. Biologische Siliermittel enthalten in der Regel Milchsäurebakterien. Chemische Siliermittel dienen zur chemischen Ansäuerung des Gärsubstrats und damit zur Unterdrückung von Gärschädlingen. Chemische Siliermittel umfassen organische und anorganische Säuren oder deren zugehörigen Salze sowie Zusammensetzungen mit einem oder mehreren dieser Stoffe.

Bevorzugte Siliermittel sind daher Ameisensäure, Formiat, Propionsäure, oder Propionat, oder enthalten zudem:
Adipinsäure, Natriumadipat, Kaliumadipat; Apfelsäure, Kaliummalat; Ascorbinsäure, Natrium-L-Ascorbat, Calcium-L-Ascorbat; Bernsteinsäure; Essigsäure, Kaliumacetat, Natriumacetat, Calciumacetat; Fumarsäure; Metaweinsäure, Calciumtartrat; Milchsäure, Lactat; Phosphorsäure, Natriumphosphat, Kaliumphosphat, Calciumphosphat; Weinsäure, Natriumtartrat, Seignettesalz (Natrium-Kalium-Tartrat); Zinn(II)-Chlorid; Zitronensäure, Natriumcitrat, Kaliumcitrat, Calciumcitrat; Sulfit; Sulfat; Nitrit; Hexamethylentetramin; Ameisensäure, Formiat; Propionsäure, Propionat; Essigsäure, Acetat; Sorbinsäure, Sorbat; Benzoesäure, Benzonat; Zitronensäure; Schwefelsäure; Salzsäure und Salpetersäure.

Die erfindungsgemäße Verwendung bezieht sich insbesondere auf die Behandlung von Zuckerrüben. In der Regel werden Zuckerrüben im Frühjahr gesät. Sie bilden im Laufe der Vegetationsperiode bis Ende September einen dicken Wurzelkörper, der den Zucker speichert. Eine Rodung der Zuckerrüben erfolgt dann ab September bis Mitte November. Die gerodeten Zuckerrüben lassen sich sodann in unzerkleinerter Form mit dem Siliermittel behandeln und bis zu ihrer späteren Weiterverarbeitung unter Luftabschluss, z.B. in Folienschläuchen, lagern.

Nach einer besonderen Ausgestaltung der Erfindung enthält das Siliermittel Formiat, insbesondere Tetraformiat

Eine andere, ebenfalls bevorzugte Ausgestaltung der Erfindung sieht vor, dass das Siliermittel Propionat enthält.

Formiate sind Salze der Ameisensäure, Propionate Salze der Propionsäure. Sofern es sich bei dem Siliermittel um eine wässrige Lösung handelt, sollte das enthaltene Salz wasserlöslich sein. Da wasserlösliche Salze von Säuren in Wasser dissoziieren und Säureanionen bilden, können anstelle der Salze auch die jeweiligen Säuren eingesetzt werden.

Die bevorzugte Konzentration von Ammoniumtetraformiat im Siliermittel liegt im Bereich von 60 - 90 Gew.-%, die von Ammoniumproprionat im Bereich von 10 - 30 Gew.-%.

Ein besonders geeignetes Siliermittel (nachfolgend "AFP" genannt) enthält
60 - 90 Gew.-% Ammoniumtetraformiat,
10 - 30 Gew.-% Ammoniumproprionat,
5 - 10 Gew.-% Milchsäure,
1 - 5 Gew.-% Glycerol sowie
0,1 - 0,5 Gew.-% Formamid.

Nach einer weiteren Ausgestaltung der Erfindung wird das Siliermittel in flüssiger Form eingesetzt. Hierbei werden die unzerkleinerten Rüben mit dem Siliermittel besprüht oder in das Siliermittel getaucht. Das Eintauchen der unzerkleinerten Rüben in das flüssige Siliermittel hat den Vorteil, dass die Rüben vollständig, das heißt von allen Seiten mit dem Siliermittel in Kontakt kommen. Nach einer kurzen Einwirkungszeit, die beispielsweise nur wenige Sekunden zu betragen braucht, werden die behandelten Rüben dann dem flüssigen Siliermittel entnommen. Anschließend erfolgt die Lagerung der Rüben.

Die Lagerung der Rüben findet unter Luftabschluss statt. Hierzu werden die Rüben in einen Behälter oder dergleichen überführt, der anschließend verschlossen wird. Durch den schnellen pH-Wert-Abfall auf der Rübenoberfläche und im Rübeninneren werden Mikroorganismen auf der Rübenoberfläche und endogene Pflanzenenzyme im Inneren der Rübenkörper weitgehend inaktiviert. Es entsteht ein stabiles System, wodurch Gesamtzucker und insbesondere Saccharose überwiegend erhalten bleibt.

Eine Lagerung kann insbesondere in verschlossenen Einrichtungen wie in Silos oder in Folienschläuchen erfolgen.

Die vorliegende Erfindung betrifft ferner ein Verfahren zur Gewinnung von Zucker aus Zuckerrüben, das die folgenden Schritte umfasst:
a. Behandlung von unzerkleinerten Zuckerrüben mit einem Siliermittel
b. Lagerung der unzerkleinerten Zuckerrüben unter Luftabschluss
c. Zerkleinerung der Zuckerrüben
d. Gewinnung von Rohsaft aus den zerkleinerten Zuckerrüben
e. Gewinnung von Zucker aus dem Rohsaft

Darüber hinaus betrifft die Erfindung ein Verfahren zur Gewinnung von Bioethanol aus Zuckerrüben. Dieses Verfahren sieht folgende Schritte vor:
a. Behandlung von unzerkleinerten Zuckerrüben mit einem Siliermittel
b. Lagerung der unzerkleinerten Zuckerrüben unter Luftabschluss
c. Zerkleinerung der Zuckerrüben
d. Gewinnung von Ethanol aus den zerkleinerten Zuckerrüben

Die obigen Ausführungen zur Zusammensetzung sowie zur Art des Einsatzes des Siliermittels gelten auch für die Verfahren zur Zucker- und Bioethanolgewinnung.

Nach der Behandlung der unzerkleinerten Zuckerrüben werden diese unter Luftabschluss, wie oben dargestellt, gelagert.

Anhand der folgenden Beispiele wird die Erfindung näher erläutert:

### Behandlung von Zuckerrüben mit einem Siliermittel

Entblattete, gewaschene, unzerkleinerte Zuckerrüben (Zuckerrübenspeicherwurzeln) wurden für wenige Sekunden in ein Bad mit dem Siliermittel AFP getaucht. Die Aufwandmenge betrug ca. 2 L/t Frischmasse.

Die unzerkleinerten Zuckerrüben wurden anschließend in Silierfässer mit einem Volumen von jeweils 120 L überführt und luftdicht verschlossen. Die Fässer bestanden aus Plastik und waren unten mit einem auf ca. 15 cm Höhe befindlichen Edelstahlboden versehen, durch den entstandenes Sickerwasser ablaufen konnte. Fermentationsgas konnte über ein aufgesetztes Gärrohr entweichen. Die Lagerung der Fässer erfolgte bei 10°C.

Nach 90 Tagen wurden die Fässer geöffnet. Von allen Rüben eines Fasses wurde eine repräsentative Rübenbreiprobe hergestellt und sofort bei -20 °C eingefroren. Die Ergebnisse der chemischen Analyse der Breiproben sind in folgender Tabelle zusammenfasst. Bei der T₀-Probe handelt es sich um eine Teilmenge der zur Behandlung und Silierung eingesetzten Rüben direkt vor der Behandlung/Einlagerung. Als Kontrolle dienten Rüben, die mit Wasser anstelle von dem Siliermittel behandelt worden waren.

| | pH | Gesamtzucker (Saccharose, Glukose und Fruktose) | Saccharose |
|---|---|---|---|
| | | in % auf Frichmasse | |
| T₀-Probe | 6,5 | 18,5 | 17,9 |
| Kontrolle | 3,5 | 5,6 | 2,5 |
| AFP | 4,0 | 17,8 | 10,7 |

Bezogen auf die jeweils vorhandene Frischmasse, bleibt die Behandlung der Rüben mit dem Siliermittel AFP ca. 95% des Gesamtzuckers bzw. ca. 60% der Saccharose erhalten.

### Zuckergewinnung aus Zuckerrüben nach Behandlung mit einem Siliermittel

Das erfindungsgemäße Verfahren zur Gewinnung von Zucker aus unzerkleinerten Zuckerrüben nutzt die Möglichkeit der Zuckerrübenlagerung, bei der im Vergleich zu einer Lagerung ohne Siliermittelbehandlung deutlich weniger Zucker verloren geht. Hierzu werden gerodete Zuckerrüben wie oben angegeben mit Siliermittel behandelt und anschließend in Silos oder in Folienschläuchen gelagert. Die so behandelten und gelagerten Zuckerrüben lassen sich anschließend je nach freier Kapazität der Zuckerfabriken weiterverarbeiten.

In Zuckerfabriken werden die Zuckerrüben von eventuell noch vorhandenem Schmutzanhang befreit und mittels Schneidmaschinen zerkleinert. Schmale Schnitzel gelangen dann in einen Brühtrog, in dem sie in heißem Wasser auf ca. 70 °C erwärmt werden. Bei dieser Temperatur werden die Zellwände der Schnitzel durchlässig, so dass der Zucker aus den Schnitzeln gewonnen werden kann. Dies geschieht in Turmanlagen, in denen die Schnitzel im Gegenstromverfahren mit heißem Wasser entzuckert werden.

Der gewonnene Rohsaft enthält neben Zucker noch sogenannte Nichtzuckerstoffe, die eine Kristallisation des Zuckers erschweren und in der Saftreinigung weitgehend entfernt werden. Zur Reinigung wird der Rohsaft mit Kalkmilch versetzt. Der zugesetzte Kalk wird anschließend durch Einleiten von Kohlensäure wieder ausgefällt und der Schlamm nach Eindickung durch Filtration vollständig abgetrennt. Durch die Saftreinigung entsteht ein klarer, hellgelber Dünnsaft.

Der Dünnsaft wird in einer mehrstufigen Verdampfstation zu Dicksaft konzentriert. Die weitere Eindickung wird in dampfbeheizten Kochapparaten bis zur Kristallbildung fortgesetzt. Nach dem Kochen wird ein Gemisch von Zuckerkristallen und anhaftendem Sirup zur Abkühlung und Fortsetzung der Kristallisation in Maischen (Gefäße mit Rührwerken) abgefüllt. In Zentrifugen erfolgt die Abschleuderung des Sirups von den Zuckerkristallen. Der gewonnene Weißzucker wird getrocknet, gekühlt und nach Absiebung von Fein- und Grobanteilen in Großraumsilos gelagert. Rohsaft, Dünnsaft und Dicksaft sind geeignete Substrate für die Ethanolherstellung durch mikrobielle Fermentation.

## Patentansprüche

1. Verwendung eines der Ansäuerung dienenden Siliermittels zur Behandlung von unzerkleinerten Rüben, um bei einer Lagerung der Rüben einen Abbau von Zucker zu reduzieren, **dadurch gekennzeichnet, dass** das Siliermittel Formiat und/oder Propionat enthält.

2. Verwendung nach Anspruch 1, **dadurch gekennzeichnet, dass** es sich bei den Rüben um Zuckerrüben handelt.

3. Verwendung nach Anspruch 1 oder 2, **dadurch gekennzeichnet, dass** das Siliermittel Tetraformiat enthält.

4. Verwendung nach Anspruch 3, **dadurch gekennzeichnet, dass** der Gehalt an Ammoniumtetraformiat 60 - 90 Gew.-% beträgt.

5. Verwendung nach Anspruch 1, **dadurch gekennzeichnet, dass** der Gehalt an Ammoniumpropionat 10 - 30 Gew.-% beträgt.

6. Verwendung nach einem der Ansprüche 1 bis 3, **dadurch gekennzeichnet, dass** das Siliermittel neben
Ammoniumtetraformiat und
Ammoniumpropionat
5 - 10 Gew.-% Milchsäure,
1 - 5 Gew.-% Glycerol sowie
0,1 - 0,5 Gew.-% Formamid
enthält.

7. Verwendung nach einem der Ansprüche 1 bis 6, **dadurch gekennzeichnet, dass** das Siliermittel in flüssiger Form eingesetzt wird, wobei die unzerkleinerten Rüben mit dem Siliermittel besprüht oder in das Siliermittel getaucht werden.

8. Verwendung nach einem der vorhergehenden Ansprüche, **dadurch gekennzeichnet, dass** die Rüben nach der Behandlung mit dem Siliermittel unter Luftabschluss gelagert werden.

9. Verwendung nach Anspruch 8, **dadurch gekennzeichnet, dass** die Lagerung der Rüben in einer luftdicht verschlossenen Einrichtung erfolgt.

10. Verwendung nach Anspruch 8 oder 9, **dadurch gekennzeichnet, dass** die Lagerung der Rüben in einem Folienschlauch erfolgt.

11. Verfahren zur Gewinnung von Zucker aus Zuckerrüben, das die folgenden Schritte umfasst:
a. Behandlung von unzerkleinerten Zuckerrüben mit einem der Ansäuerung dienenden Siliermittel
b. Lagerung der unzerkleinerten Zuckerrüben unter Luftabschluss
c. Zerkleinerung der Zuckerrüben
d. Gewinnung von Rohsaft aus den zerkleinerten Zuckerrüben
e. Gewinnung von Zucker aus dem Rohsaft,
**dadurch gekennzeichnet, dass** das Siliermittel Formiat und/oder Propionat enthält.

12. Verfahren zur Gewinnung von Bioethanol aus Zuckerrüben, das die folgenden Schritte umfasst:
a. Behandlung von unzerkleinerten Zuckerrüben mit einem der Ansäuerung dienenden Siliermittel
b. Lagerung der unzerkleinerten Zuckerrüben unter Luftabschluss
c. Zerkleinerung der Zuckerrüben
d. Gewinnung von Ethanol aus den zerkleinerten Zuckerrüben,
**dadurch gekennzeichnet, dass** das Siliermittel Formiat und/oder Propionat enthält.

13. Verfahren nach Anspruch 11 oder 12, **dadurch gekennzeichnet, dass** das Siliermittel Tetraformiat enthält.

14. Verfahren nach Anspruch 13, **dadurch gekennzeichnet, dass** der Gehalt an Ammoniumtetraformiat 60 - 90 Gew.-% beträgt.

15. Verfahren nach einem der Ansprüche 11 oder 12, **dadurch gekennzeichnet, dass** der Gehalt an Ammoniumpropionat 10 - 30 Gew.-% beträgt.

16. Verfahren nach einem der Ansprüche 11 bis 13, **dadurch gekennzeichnet, dass** das Siliermittel neben
Ammoniumtetraformiat und
Ammoniumpropionat
5 - 10 Gew.-% Milchsäure,
1 - 5 Gew.-% Glycerol sowie
0,1 - 0,5 Gew.-% Formamid
enthält.

17. Verfahren nach einem der Ansprüche 11 bis 16, **dadurch gekennzeichnet, dass** das Siliermittel in flüssiger Form eingesetzt wird, wobei die unzerkleinerten Zuckerrüben mit dem Siliermittel besprüht oder in das Siliermittel getaucht werden.

## Claims

1. Use of an acidifying ensiling agent for the treatment of unchopped beets to reduce degradation of sugar occurring during storage of the beets thereby characterized that the ensiling agent comprises formate and / or propionate.

2. The use according to claim 1, thereby **characterized in that** the beets are sugar beets.

3. The use according to claim 1 or 2, **characterized in that** the ensiling agent comprises tetraformate.

4. The use according to claim 3, thereby **characterized in that** the content of ammonium tetraformate is 60 to 90 weight -%.

5. The use according to any one of claim 1, thereby **characterized in that** the content of ammonium propionate is 10 - 30 weight -%.

6. The use according to any one of claims 1 to 3, thereby **characterized in that** the ensiling agent comprises beside to
ammonium tetraformate and
ammonium propionate
5 - 10 wt -% lactic acid,
1 - 5 wt -% glycerol as well as
0.1 to 0.5 wt -% formamide.

7. The use according to one of the claims 1 to 6, thereby **characterized in that** the ensiling agent is used in liquid form, wherein the unchopped sugar beets are sprayed with the ensiling agent or dipped into the ensiling agent.

8. The use according to one of the preceding claims, thereby **characterized in that** the storage of the beets occurs under anaerobic conditions.

9. The use according to claim 8, thereby **characterized in that** the storage of the beets occurs in an airtight sealed device.

10. The use according to claim 8 or 9, thereby characterized that the storage occurs in a foil tube.

11. A method for extraction of sugar from sugar beets, comprising the steps of:
a. treatment of unchopped sugar beets with an ensiling agent
b. storage of the unchopped sugar beets under anaerobic conditions
c. chopping the sugar beets
d. extraction of raw juice from the chopped sugar beets
e. extraction of sugar from the raw juice,
thereby characterized that the ensiling agent comprises formate and / or propionate.

12. A method for production of bioethanol from sugar beets, comprising the following steps:
a. treatment of unchopped sugar beets with an ensiling agent
b. storage of the unchopped sugar beets under anaerobic conditions
c. chopping the sugar beets
d. producing ethanol from the chopped sugar beets,
thereby characterized that the ensiling agent comprises formate and / or propionate.

13. The method according to claim 11 or 12, thereby **characterized in that** the ensiling agent comprises tetraformate.

14. The method according to claim 13, thereby **characterized in that** content of ammonium tetraformate is 60 to 90 weight -%.

15. The method according to any one of claims 11 or 12, thereby **characterized in that** content of ammonium tetraformate is 10 to 30 weight -%.

16. The use according to one of claims 11 to 13, thereby **characterized in that** the ensiling agent comprises beside to
ammonium tetraformate and
ammonium propionate
5 - 10 wt -% lactic acid,
1 - 5 wt -% glycerol as well as
0.1 to 0.5 wt -% formamide.

17. The method according to any one of claims 11 to 16, thereby **characterized in that** the ensiling agent is used in liquid form, wherein the unchopped sugar beets are sprayed with the ensiling agent or dipped into the ensiling agent.

## Revendications

1. Utilisation d'un agent d'ensilage servant à l'acidification, pour le traitement de tubercules entiers, pour réduire une dégradation du sucre lors du stockage des tubercules, **caractérisée en ce que** l'agent d'ensilage contient un formiate et/ou un propionate.

2. Utilisation selon la revendication 1, **caractérisée en ce que** les tubercules sont des betteraves.

3. Utilisation selon la revendication 1 ou 2, **caractérisée en ce que** l'agent d'ensilage contient un tétraformiate.

4. Utilisation selon la revendication 3, **caractérisée en ce que** la teneur en tétraformiate d'ammonium s'élève à de 60 à 90 % en poids.

5. Utilisation selon la revendication 1, **caractérisée en ce que** la teneur en propionate d'ammonium s'élève à de 10 à 30 % en poids.

6. Utilisation selon l'une quelconque des revendications 1 à 3, **caractérisée en ce qu'**en plus
du tétraformiate d'ammonium et
du propionate d'ammonium,
l'agent d'ensilage contient
de 5 à 10 % en poids d'acide lactique,
de 1 à 5 % en poids de glycérol ainsi que
de 0,1 à 0,5 % en poids de formamide.

7. Utilisation selon l'une quelconque des revendications 1 à 6, **caractérisée en ce qu'**on utilise l'agent d'ensilage sous forme liquide, sachant qu'on vaporise les tubercules entiers avec l'agent d'ensilage ou on les plonge dans l'agent d'ensilage.

8. Utilisation selon l'une quelconque des revendications précédentes, **caractérisée en ce qu'**après le traitement avec l'agent d'ensilage, on stocke les tubercules sous exclusion d'air.

9. Utilisation selon la revendication 8, **caractérisée en ce que** le stockage des tubercules s'effectue dans une installation fermée de manière étanche à l'air.

10. Utilisation selon la revendication 8 ou 9, **caractérisée en ce que** le stockage des tubercules s'effectue dans un film tubulaire.

11. Procédé destiné à extraire du sucre de betteraves, qui comprend les étapes suivantes :
a. Traitement de betteraves entières avec un agent d'ensilage servant à l'acidification
b. Stockage des betteraves entières sous exclusion d'air
c. Broyage des betteraves
d. Extraction de jus brut des betteraves broyées
e. Récupération de sucre à partir du jus brut
**caractérisé en ce que** l'agent d'ensilage contient un formiate et/ou un propionate.

12. Procédé destiné à extraire du bioéthanol de betteraves, qui comprend les étapes suivantes :
a. Traitement de betteraves entières avec un agent d'ensilage servant à l'acidification
b. Stockage des betteraves entières sous exclusion d'air
c. Broyage des betteraves
d. Extraction d'éthanol des betteraves broyées,
**caractérisé en ce que** l'agent d'ensilage contient un formiate et/ou un propionate.

13. Procédé selon la revendication 11 ou 12, **caractérisé en ce que** l'agent d'ensilage contient un tétraformiate.

14. Procédé selon la revendication 13, **caractérisé en ce que en ce que** la teneur en tétraformiate d'ammonium s'élève à de 60 à 90 % en poids.

15. Procédé selon l'une quelconque des revendications 11 ou 12, **caractérisé en ce que** la teneur en propionate d'ammonium s'élève à de 10 à 30 % en poids.

16. Procédé selon l'une quelconque des revendications 11 à 13, **caractérisé en ce qu'**en plus
du tétraformiate d'ammonium et
du propionate d'ammonium,
l'agent d'ensilage contient
de 5 à 10 % en poids d'acide lactique,
de 1 à 5 % en poids de glycérol ainsi que
de 0,1 à 0,5 % en poids de formamide.

17. Procédé selon l'une quelconque des revendications 11 à 16, **caractérisé en ce qu'**on utilise l'agent d'ensilage sous forme liquide, sachant qu'on vaporise les tubercules entiers avec l'agent d'ensilage ou on les plonge dans l'agent d'ensilage.
